# EUROPÄISCHE PATENTANMELDUNG

(11) **EP 1 839 678 A2**
(43) Veröffentlichungstag der Anmeldung: **03.10.2007**
(21) Anmeldenummer: 07006200.5
(22) Anmeldetag: 27.03.2007
(51) Int. Cl.: A61L 2/03

(54) **Vorrichtung und Verfahren zur selektiven Abreicherung, Inaktivierung, Stimulierung, Elimination oder Lyse von biologischen Partikeln -in-vitro / in-vivo - in biologischen-, physiologischen- und industriellen Flüssigkeiten**

(30) Priorität: 29.03.2006 EP 06006482
(71) Anmelder: Pautz, Norbert, 5412 Puch (AT)
(72) Erfinder: Pautz, Norbert, 5412Puch (AT)

(57) **Zusammenfassung**

Die Erfindung betrifft ein physikalisches Verfahren mit einer Vorrichtung, wo eine Flüssigkeit mit einem definierten Fluss durch eine biokompatible Apparatur geleitet wird, die eine gezielte, gleichzeitige Behandlung durch physikalische Effekte wie Elektrolyse, Elektrophorese, Elektroporation, spannungs- / stromstärke- abhängiges Öffnen von Zellen (reversibel) und Oszillation / Resonanz durch Pulsung von Gleichstrom mit variabler Frequenz- und Pulsweitenmodulation, angeregte und letale Zustände von Zellen erzeugt, wobei chemische Verbindungen die beispielsweise durch Elektrolyse erzeugt wurden, leichter zur Reaktion gebracht werden. Die genannten Prozesse laufen dynamisch parallel oder überlagernd sowie auch hintereinander ab, wobei je nach Zell-Art und dem umgebenden, flüssigen Medium eine Abstimmung erfolgt, welcher Prozess wie z.B. die Elektrolyse primär bzw. vorrangig erfolgt.

Durch Wahl und Steuerung der zur Verfügung stehenden Parameter oder deren Kombination, ist es möglich eine Selektion herbeizuführen, um bestimmte Zellen bzw. biologische Partikel z.B. Bakterien, Viren, Phagen - in-vitro / in-vivo - in einer Flüssigkeit zu inaktivieren, abzureichern, zu stimulieren oder zu lysieren oder partiell zu oxidieren. Das Verfahren ist skalierbar.

## Beschreibung

Die Erfindung betrifft ein physikalisches, dynamisches Verfahren und eine Vorrichtung, wo eine Flüssigkeit mit einem definierten Fluss durch eine Apparatur geleitet wird, wo zeitlich gesteuert, eine Behandlung von biologischen Partikeln durch gezielte, unterschiedliche aber parallele, physikalische Effekte angeregte und / oder letale Zustände erzeugt werden.

**Definition-:**
*Biologischen Flüssigkeiten* sind Körperflüssigkeiten animalischer oder humaner Herkunft, wie Voll-Blut, Blut-Serum, Blut-Plasma oder Blut- und Plasma-Präparate (Plasma-Ersatzstoffe), Vakzine weiterhin auch eine Suspension / Lösungen von monoklonalen Antikörpern oder eine Suspension von Stammzellen.
*Physiologische Flüssigkeiten* sind Infusionslösungen, Injektionslösungen als Arzneimittel-Lösung, Elektrolyt-Lösungen in Mischung oder als Einzel-lon (NaCl) die in einer bestimmten, physiologischen Konzentration vorliegen, sowie Lösungen zur parenteralen und enteralen Ernährung wie Vitamin-Lösungen, Aminosäure- oder Zuckerlösungen oder aber auch andere Nährstofflösungen wie auch Fermentationslösungen.
*Industrielle Flüssigkeiten* sind Suspensionen, Emulsionen oder Lösungen auf Wasserbasis, die in verschiedenen industriellen Bereichen gefunden werden, so z.B. in der Getränkeindustrie wie Bier, Limonaden, Fruchtsäfte, Tee- oder Kaffee-Getränke oder in der Lebensmittelindustrie wie z.B. Milch, Roh-Milch, Milch-Getränke (Mix-Getränke), Milchserum. Andere industrielle Flüssigkeiten beziehen sich auf technische Anwendungen wie bei der spanabhebenden Fertigung die Kühlschmierstoffe oder Kühlmittel bevorzugt auf Wasserbasis.
*Biologische Partikel* können sein: Bakterien, Sporen, Viren, Phagen, Prionen, Parasiten, Protozoen, Pollen und tierische Zellen, humane Zellen oder auch pflanzliche Zellen aller Art.
*Biokompatible Apparatur* ist eine Apparatur durch die eine biologische Flüssigkeit strömt deren Bauteile bzw. Bestandteile aus Werkstoffen besteht, die keine negativen (toxischen) Einflüsse auf Lebewesen haben, da keine Bestandteile des Werkstoffs an die Flüssigkeit abgegeben werden oder der Werkstoff selbst nicht mit der biologischen Flüssigkeit reagieren kann oder in Wechselwirkung tritt.

### Stand der Technik

Für die Inaktivierung / Lyse von resistenten Bakterien und Viren / Phagen sind folgende Techniken geeignet:
- Chemische Methoden (Detergenzien, alkalische Lyse, Protease-Inhibitoren, Biozide u.a. Pharmaka)
- Physikalische Methoden (UV-Licht, Erhitzen, Dampf-Sterilisation, Nano-Partikel Filtration / Adsorbtion, Ultra-Filtration, Elektroporation, Ultraschall, Elektrolyse u.a.)
Bei derartigen Verfahren, die eine Form von Wechselwirkungen von magnetischen-, elektrischen- bzw. elektromagnetischen Feldern in Flüssigkeiten auf biologische Partikel nutzen, hat man in konduktiven Medien, wenn ein Strom fliesst, ebenso elektrolytische oder elektrophoretische als auch andere Effekte, die Auswirkungen auf die Matrix - das fliessende, flüssige Medium - oder auch auf biologische Partikel bzw. Zellen direkt, aufweisen - nur werden diese Einzeleffekte nicht gezielt genutzt.
Das U.S: Patent 6,539,252 (25.März, 2003) beschreibt eine Elektrolyse mit einer Silber-Elektrode die zur Inaktivierung von "Blut-Pathogenen" Siber-Kationen freisetzt die auch Viren inaktivieren soll.
Das U.S. Patent 7,029,916 (18 April, 2006) beschreibt die Elektroporation von Zellen im Blut, das Stimulieren der Zellen um biologisch, aktive Substanzen frei zu setzen oder besser aufzunehmen.
Das U.S. Patent 5,931,802 (3 August, 1999) beschreibt die Elektrolyse als "Extrakorporaler-Blut-Zirkulator".
Das U.S. Patent 6,074,605 (13 Juni, 2000) beschreibt eine Elektroporations-Methode und Kammer, wo Gleichstrom gepulst und von unterschiedlicher Frequenz und Polarität eingesetzt wird zur therapeutischen, positiven Änderung der physikalischen Charakteristik von intrazellulärem Hämoglobin.
Die physikalischen Grundlagen der Elektroporation sind bekannt und werden bereits in Geräten-Systemen die frei am Markt erhältlich sind - Beispiel: Multiporator® der Firma Eppendorf eingesetzt (allerdings statisch in kleinen Volumen / ml-Bereich).

Ein weiteres U.S. Patent 5,139,684 (18 August, 1992) beschreibt den Einsatz einer elektrischen, konduktiven Methode zur Behandlung von Blut oder anderen Körperflüssigkeiten mit elektrischen Feldern bzw. Feldstärken. Es wird hier angegeben, HIV wirksam inaktiviert, zu haben. Es wird ausgeführt dass durch Anlegen einer Spannung mittels Platin-Elektroden (0.2 bis 12 Volt) bei einer Stromstärke im Micro-Ampere bis Milli-Ampere-Bereich innerhalb einer Behandlungszeit von 1 bis 12 Minuten HIV inaktiviert wurde. Ein konduktiv leitender Behälter mit einer Ausbildung von weiteren konduktiv leitenden Röhren mit unterschiedlichen Anordnungen wird von Blut durchströmt und mit Hilfe elektrische Feldstärken das Virus inaktiviert. Die Spannung wird im Puls-Mode mit Gleichstrom durchgeführt.
Es sind weiter folgende Patentanmeldungen bekannt: USA - 2002168456 ; hier wird gepulster Gleich-Strom bei 120 Hz und einer Amplitude von 6 bis 15 Volt - eingesetzt. Als Kathode wird Edelstahl und als Anode eine Wolfram / Kupfer Legierung (75% zu 25%) eingesetzt. Hier wurden diverse Bakterien in Fruchtgetränken abgetötet.
US-A-5348629 beschreibt ein statisches Verfahren eines elektrochemischen Prozesses einer Elektrolyse wo primär Oxidation und Reduktion, Dimerisation, Polymerisation organischer Verbindungen auch für Koagulations-Prozesse bzw. Sammeln und Trennen von Suspensionen, Emulsionen oder auch Inaktivierung und Zerstörung von Mikroorganismen erfolgen. Verfahren und Apparatur für elektrolytische Prozesse von Materialien werden beschrieben.

US-A 3625843 beschreibt ein Verfahren zur Reduktion oder Elimination von Mikroorganismen in Substanzen durch elektrische Stimulation. Diese Erfindung befasst sich mit der Stimulation hoch energetischer Felder in Wellenform, gepulst bei hohen Stromstärken und Stromdichten unter möglichem Zusatz von Edel-MetallIonen, bei Durchsatz von Feststoffen oder auch Flüssigkeiten durch eine Elektrodenkammer in rechtwinkliger Anordnung wobei zwei grosse Elektroden-Platten gegenüber angeordnet sind, die im Gleichstrom bei 12 bis 100 Volt mit Platinelektrodenplatten betrieben werden - ohne Polaritätswechsel. Es erfolgt eine Pulsung im positiven (+) Bereich, wobei zum Schluss, um die Kathode zu depolarisieren ein negativer Puls erfolgt. Es handelt sich um keine Elektrolyse.
Offenlegungsschrift DE 32 33 282 A1 beschreibt ein Verfahren der Anwendung von Nieder- oder Hochfrequenzfeldern zur thermischen und nicht thermischen Inaktivierung von Mikro-Organismen - auch unter einer Gasentladung, die Ozon erzeugt. Dieses Verfahren ist keine Elektrolyse.
US-Patent 3,625,843 beschreibt eine sehr spezifische Anwendung von einer Art von elektromagnetischen Pulsen die mit Kohlenstoff (Carbon) Anoden- und Kathoden durchgeführt wird, wobei der Aufbau der Vorrichtung in Kaskadenform sehr spezifisch ist und eine Flüssigkeit in der Anwendung Bier, hier pasteurisiert bzw. sterilisiert wird. Durch die Anordnung von Kathode und Anode wird eine Art von Kondensator mit kapazitiver Wechselwirkung erreicht, welche die metabolische Aktivität von Mikro-Organismen stoppen soll, da der lonen-Austausch verändert wird.

PCT-Anmeldung WO 99/08719 bezeichnet eine Erfindung eines Sterilisations-Apparates unter Verwendung der Elektrolyse von Wasser bzw. Salzlösung. Es handelt sch hier um die alte "Faraday'sche Elektrolyse" mit Hilfe eines Diaphragmas. Es werden zwei Fraktionen - anodisch / "anolyte" (sauer) und "kathodisch" / catholyte (basisch). Unter Zusatz von Detergenzien wird in einem geschlossenen System eine Sterilisation durchgeführt.
DE 20 2004 010 572 U1 -zeigt die Apparatur eines hygienischen Wasserspenders. Auch hier handelt es sich um die bekannte Ausführung der Elektrolyse mit einem Diaphragma aus Keramik. Es handelt sich hierbei um eine spezifische Anordnung von Bauteilen um den Wasserspender zu gestalten. Allerdings wird hier eine Titan-MOX-Anode verwendet.
EP 0949 323 A2 - zeigt ein Verfahren zur Verlängerung der Gebrauchsfähigkeit eines Kühlschmierstoffes. Hier wird "Knallgas" produziert. Knallgas entsteht durch eine "explosionsartige", exotherme Reaktion von Sauerstoff mit Wasserstoff. Diese werden bei der Elektrolyse von Kühlschmierstoff der zu etwa 95% aus Wasser besteht gebildet. An der Kathode steigt Wasserstoff auf und anodisch entsteht Sauerstoff. Durch ein Beladen von feinen Gasblasen mit Fremdöl und Schmutzpartikelchen wird im Flotations-Prinzip, die Kühlschmierstoff-Emulsion gereinigt. Atomarer Sauerstoff soll die Abtötung von Keimen erledigen und ein anderer Teil von Mikroorganismen soll bei Kontakt an den Elektroden eliminiert werden. Bei einem Einsatz von Titan mit Mischoxiden beschichteten oder platinbeschichteten Elektroden bei Strömungen von 300 I pro Stunde bei 4 Ampere und einer Spannung zwischen 2 und 48 Volt Gleichstrom und "gelegentlicher" Umpolung. Eine Depotwirkung wird erzielt durch im Wasseranteil gelöster Chlorid-Ionen usw. Das Verfahren funktioniert nicht, da durch grössere Gasmengen fein verteilter Gasblasen jede Emulsion destabilisiert wird (Stand der Technik) - nach den Angaben geschieht keine Elektrolyse. Chlorid-Ionen allein im Kühlschmierstoff verhindern kein Wachstum von Mikroorganismen. Da primär ein Gas hier "atomarer" Sauerstoff erzeugt werden soll, muss die Elektroden-Art darauf optimiert werden. Ebenso muss EMK der Zelle und somit die katalytische Aktivierung mit dem Arbeitspotenzial abgestimmt und optimiert werden, damit überhaupt Desinfizenzien gebildet werden können. Das ganze beschriebene Verfahren ist konfus, und nicht nach den Erkenntnissen der Wissenschaft formuliert (Stand der Technik). Um atomaren Sauerstoff (gemeint sind wohl Sauerstoff-Radikale) darzustellen bedarf es ganz anderer Bedingungen. Das Verfahren kann so keine signifikanten Keimreduktionen herbeiführen - es ist nach den Unfallverhütungsvorschriften, zu verbieten, da Explosionsgefahr besteht (Knallgas).
Eine weitere Patentschrift - DE 44 08 797 A1 - beschreibt ein Verfahren und Vorrichtung zur Stabilisierung von Kühlschmierstoffen. Durch Anheben des pH-Wertes bei der Entfernung von Hydrogenkarbonat mit Hilfe des elektrischen Stromes
- also Elektrolyse - mit einem Diaphragma oder Membrane. Es wird ein Kühlschmierstoff im pH-Bereich von 8 bis 9 angehoben. Das Verfahren funktioniert nicht und ist ungeeignet, da Mikroorgnismen über den gesamten pH-Bereich ubiquitär vorhanden sind. Eine signifikante Reduktion ist überhaupt nicht erreichbar. Eine Elektrolyse mit Diaphragma / Membrane spaltet den Kühlschmierstoff und ist ungeeignet für eine wirksame Entkeimung - der KSS wird gespalten. Technisch ist dieses nicht durchführbar, da Metall-und Schmutzpartikel die Membrane zusetzen also verstopfen, da Kühlschmierstoff offen in der Metallbearbeitung eingesetzt wird.
- Allein eine Änderung des pH-Wertes ergibt keine signifikante Reduktion des Wachstums von Mikroorganismen.
Eine weitere Patentschrift ist das EP 0 862 538 B1 - Verfahren und Vorrichtung zur Behandlung von mit Mikroorganismen und/ oder Schadstoffen belastetem Wasser. Hier handelt es sich um eine echte Fluss-Elektrolyse ohne Diaphragma die aber auf die Verwendung von Titan-Titandioxid-Elektroden beschränkt ist. Sie erfolgt im Niedervoltbereich unter Beibehaltung der Polarität. Die Elektroden-Gestaltung war als Gitter mit ringförmiger Anordnung oder parallel als Plattenanordnung ausgebildet. Es gibt noch eine Vielzahl von Publikationen und Patentanmeldungen in diesem Bereich die nicht alle diskutiert werden können.

Diese Verfahren sind entweder sehr spezifisch auf eine Applikation ausgerichtet oder weisen entsprechende Nachteile auf. Schaut man sich in Praxis um bei den grössten Getränkeherstellern findet man keine Elektrolyse, die Getränke entkeimt. Das gleiche gilt für Milch bei den weltweit führenden Herstellern. Auch ein Dekontaminieren von Körperflüssigkeiten wie Blut wird nirgends durchgeführt, ist aber wie andere Verfahren, in Patentanmeldungen beschrieben worden.
Dieses hat offensichtlich wirtschaftliche Gründe, da das vorgeschlagene Verfahren zu teuer im technischen Massstab ist oder es hat Umsetzungs-Probleme die keine gute technische Lösung erlauben oder was oft vorkommt - es funktioniert überhaupt nicht.
Im vorliegenden Fall wo es um Flüssigkeiten geht, ist nach den Kriterien die Matrix (Trägerflüssigkeit) selbst und den darin befindlichen biologischen Partikeln zu differenzieren und zu optimieren. Die Fragestellung, ob Bakterien, Hefen oder Pilze entfernt werden sollen oder gar Viren oder Phagen spielt eine entscheidende Rolle.
Bei den thermischen Verfahren oder chemischen Verfahren findet in der Regel ein "Over-Kill" statt. Es werden zu hohe Temperaturen angewendet, was wenig effizient für den Energie-Einsatz ist oder chemische Stoffe, z.B. Biozide werden zu hoch dosiert, so dass eine Beeinträchtigung des Menschen oder der Umwelt eintritt, da diese Stoffe meistens nicht leicht biologisch abbaubar sind. Ein anderer Nachteil ist die Resistenz-Bildung von Mikroben. Auch werden bei thermischen Verfahren Sporen teilweise nicht inaktiviert. Ein weiterer Grund ist die Toxizität von chemischen Stoffen. Es gibt eine Vielzahl anderer Techniken zur Entkeimung wie Gammastrahlung, Begasung, UV-Licht usw. die aber nicht weiter diskutiert werden. Es geht hier primär um die Technologie der Elektrolyse oder auf Einsatz der durch Strom erzeugten Wechselwirkungen.

Chemische Methoden und Verfahren weisen aber bestimmte Nachteile auf, und sind bisher nicht zur Inaktivierung von resistenten Keimen und Viren einsetzbar vor allen Dingen nicht in Voll-Blut - in-vivo / in vitro - im humanen Bereich. Ein anderes Beispiel sind Netzer bzw. Detergenzien. Diese sind nicht immer genug spezifisch und brechen oft alle Zellen (es solubilisieren Membranproteine) auf und sind weiterhin auch schwer wieder, zu entfernen. Es gelingt auch heute kaum quantitativ Bakterien und Hefen nebeneinander mit einem Detergens zu "öffnen" um mikrobiologisches ATP zu extrahieren.
Bis heute gibt es kein Verfahren, dass im Vollblut ohne Neben-Effekte resistente Bakterien und Viren entfernt.

Ein physikalisches Verfahren, das oft angetroffen wird, ist die Entkeimung durch UV-Strahlung. Derartige Verfahren sind teilweise schwer beherrschbar, da bei einer harten UV-Strahlung im Bereich von 190 bis 254 nm oft nicht nur Mikroorganismen, sondern auch das herzustellende Protein geschädigt wird, da die UV-Strahlung unspezifisch, nicht selektiv arbeitet. Man kann beispielsweise, wenn eine Phagen-Infektion in der Kultur von E. coli vorliegt, nicht die Phagen selektiv entfernen (abreichern).

Die Kritik am Stand der Technik kann dahingehend weitergeführt werden, dass vorgeschlagene Verfahren teilweise zu unselektiv sind und andere Stoffe oder biologische Zellen im Gesamtsystem nachteilig beeinflussen. Sehr viele Verfahren nutzen die Möglichkeit eines gepulsten Gleichstroms im Nieder-Volt- oder auch Hoch-Volt-Bereich. Gepulster Strom führt bei fast allen Elektroden-Typen zur Erwärmung. Auch die Haltbarkeit bzw. Lebensdauer der Elektroden ist wesentlich kürzer. Selten wird Wechselspannung direkt angewendet. Auch bei gepulster Arbeitsweise einer Elektrolyse kann eine Driften des pH-Wertes eintreten. Bei früheren Patentveröffentlichungen fällt auf, dass keine moderne Elektroden-Technologie eingesetzt wird.

So ist die von Kaali et. al. (US-Patent 5,139,684) vorgeschlagene Behandlung von Körperflüssigkeiten durch elektrische- , magnetische- oder elektromagnetische Felder zur Inaktivierung von HIV mit Platinelektroden oder Stromfluss durch elektrisch leitfähigen Kunststoff mit röhrenförmiger Anordnung nicht zu Ende gedacht, da bei dem Stromfluss 0,2 bis 12 Volt entsprechend der hohen Leitfähigkeit von Blut es zur Gasbildung kommt. Das Blut schäumt stark bei wenig erhöhter Stromstärke - apparativ müsste zwingend eine Entgasung vorgesehen werden -diese fehlt völlig. Auch sind die angegebenen Parameter wie Stromstärke im Micro-Ampere-Bereich kaum messbar und die Abtötungs-Kinetik oder Inaktivierungskinetik in der beschrieben Zeitspanne bis zu 12 Minuten uneffektiv. Der HIV-Titer bzw. der Level kann so nicht gesenkt werden. Es bedarf zusätzlich anderer Lösungen da die Nebenwirkungen (Schädigung anderer Zelltypen) in der Matrix diese Technologie überfordern. Generell ist Elektrolyse allein kein probates Mittel, um Vollblut von Mikroorganismen / Viren, zu befreien.

Es können kleine Anteile, je nach physikalischen Bedingungen, von Wasserstoff, Sauerstoff und Chlor entstehen. Dieses geschieht auch, allerdings vermindert, bei dem Einsatz von gepulstem Gleichstrom. Die an den Elektroden angelegte Gleichspannung sollte die sogenannte "Überwindungs-Spannung" gegen die elektromotorische Kraft der Zelle z. B. bei Platinelektroden nicht wesentlich überschreiten, so dass die Gasbildung an der Anode durch Elektrolyse gering bleibt. Was hieraus abgeleitet werden kann, ist die Auswahl von Elektrodenmaterial mit einem Arbeitspotential, das zu den physikalischen und biologischen Parametern des Gesamtsystems - in diesem Falle Voll-Blut - passt.

Weiterhin ist bei der Inaktivierung des HIV-Virus wie in U.S. Patent 5,139,684 beschrieben durch Einfluss der Feldstärke erfolgt und nicht durch Elektrolyse. Das hier untersuchte Virus HIV bzw. HIV1 ist ein behülltes Virus der Familie der Retroviridae, Gattung Lentivirus.
Behüllte Viren sind leichter zugänglich für Anwendungen der Elektrolyse oder Applizierung von nur elektromagnetischen Feldern. Der genau Mechanismus wurde im oben genannten Patent nicht beschrieben allerdings wurde eine Inaktivierung festgestellt, die dadurch hervorgerufen wurde, dass der Proteinmantel beeinträchtigt wurde und zwar in der Weise, dass kein Enzym ("Reverse Transkriptase") mehr produziert wurde, was absolut notwendig ist, um in eine humane T-Zelle einzudringen zur Replikation.
Diese steht allerdings teilweise im Widerspruch zu Publikationen, wo durch elektrolysiertes Wasser Viren (HIV) inaktiviert wurden (Journal of Virological Methods 85 (2000) 163-174 HBV/HIV). Beide Mechanismen sind möglich. Allerdings wurden in diesem Beispiel (Kaali et. al.) sehr geringe Stromstärken angegeben bei denen der Effekt der Elektrolyse zurückgedrängt wurde und kaum sichtbar ist.

Es ist allerdings davon auszugehen, dass sehr oft Effekte parallel ablaufen oder sich überlagern können. Allerdings wurde eine gezielte Kombination von unterschiedlichen physikalischen Effekten, bewusst gesteuert, noch nicht beschrieben. Geht man zurück auf eine Forderung zur Abreicherung von resistenten Bakterien in Blut stellt sich zuerst die Frage nach den physikalischen und chemischen Parametern der Matrix. Diese sind Osmolarität, Leitfähigkeit, Dichte, Zellart und Anteil von unterschiedlichen Zellen, Bestandteile wie Wasser, Art der Elektrolyte, Proteine, Kohlehydrate, Lipide, diverse Metabolite usw.
Humane- und tierische Zellen haben einen anderen Aufbau als Bakterienzellen. Der grösste Unterschied liegt für die Applizierung von elektrischen- magnetischen bzw. elektromagnetischen Kraftfeldern oder dem Vorgang einer Elektrolyse darin, dass bei Bakterien eine Zellwand die äussere Hülle bildet gefolgt von der Zell-Membran. Säugetierzellen haben nur eine Zellmembrane. Der Unterschied in der Dicke der Zellwand und der Zellmembrane ist markant - 5 bis 10 nm für die Membrane aber etwa 200 nm Dicke für die Zellwand. Dieser Unterschied allein ist aber nicht ausschlaggebend für die Funktion der Zellwand als lonen-Austausch-Membrane.

Ein Ion-Austausch erfolgt auf beiden Seiten der Zellwand, wenn eine elektrische Spannung angelegt wird oder ein starkes magnetisches Feld erzeugt wurde. Vereinfacht dargestellt heisst dieses ein lonenaustausch findet statt was profunde Änderungen bei der chemischen Umgebung (intrazellulär und extrazellulär) wie im pH-Bereich bedeutet. Bei diesem Vorgang werden die Proteine der Zellwand und der Zellmembran beeinflusst, d.h. sie denaturieren, depolarisieren oder verlieren ihre Hydrogen-Bindung was zur Änderung der Strukturen führt. Das bedeutet das die Zelle nicht mehr metabolisch aktiv ist und Substrat umsetzen kann und im weiteren Schritt die Zellwand geschwächt wird und Risse erhält um sich als Zelle bzw. Zellverband, aufzulösen (Lyse).
Es ist eine bekannte Methode in der Flow-Cytometrie mit einem lipophilen Membran-Farbstoff physiologische Zustände wie Stress, Vitalität, "Zellschlaf"(dormant) und
Zelltod zu charakterisieren. Die Messung des Membran-Potenzials ist lange bekannt und also möglich.

Um eine Zelle oder biologischen Partikel durch elektrische Impulse oder Kraftfelder, zu beeinflussen, kann man verschiedene Möglichkeiten in Betracht ziehen:
- Elektroporation
- Spannung abhängiges Durchlassen (Voltage Dependent Gating - DVG)
- Oszillation
- Elektrolyse
- Elektrophorese
Die Elektroporation regt aufgrund von sehr kurzen (15 bis 100 Mikro-Sekunden) und schnellen Impulsen die Zellen zur Permeation (reversibel) an. Bei einer Pulslänge von 15 µS bis 100 µS werden die Membranen von tierischen und pflanzlichen Zellen durchlässiger, so dass Poren entstehen. Die Elektroporation arbeitet allerdings in Medien mit geringer Osmolarität bei exponentiell abnehmender Spannung. Die Elektroporation wurde bisher nur als statische Methode in kleinen Volumina eingesetzt. Bei wässrigen Flüssigkeiten mit einer Leitfähigkeit kann unter den Bedingungen der Elektroporation Lyse der Zellen eintreten.
Eukaryotische Zellen bauen einen Na+/K+ -Gradienten entlang der Zellmembran auf. Werden z.B. in PBS mit hoher Leitfähigkeit Na+-lonen in die Zelle geschleust kommt es zur Störung - bei hohem Stromfluss wird die Zelle irreversibel geschädigt.

Generell wird eine Zelle durch eine kritische Schwingung, bei einer bestimmten Frequenz und Energie zerstört das bedeutet letale Oszillation.
Ein spannungsabhängiges "Gating" ermöglicht Protein-Molekülen durch die Zellmembrane zu wandern - wie ein Korkenzieher. Dieses ruft "Ionen-Brücken" hervor und unter dem Einfluss eines elektrischen Potenzials werden lonen über die Zell-Membrane geführt. Dieser Mechanismus ist bei vielen "Sub-Systemen" zu finden - ein bekanntes Beispiel sind die Nervenzellen. Auch diese Methode wurde bisher nur im statischen Verfahren angewendet. Nutzung dieses Effektes im technischen Massstab (skalierbar) ist bisher unbekannt und wurde nicht eingesetzt.
Im Falle eines Bakteriums kann die Oszillation, das spannungsabhängige Durchlassen (VDG) unterdrücken und zu einer Störung der "lonen-Balance" führen, welche sich als osmotische Druckdifferenz auswirkt und letztendlich zum "Explodieren" des Bakteriums bzw. der Zelle führt. Oszillation kann technisch auf verschiedene Weisen erzeugt werden. Eine bekannte Methode die auch im Durchflussbetrieb eingesetzt wird ist durch Erzeugung von Ultraschall.

Die Elektrolyse produziert je nach Arbeitspotenzial der Elektrode und Art der Elektrode bei Anlegung einer Spannung bzw. Stromflusses bestimmte chemische Verbindungen, die aufgrund einer chemischen Reaktion, die Bakterienzelle inaktiviert, nicht aber in jedem Fall lysiert.
Dieses kann ebenso bei den Viren ablaufen wobei unspezifische Kettenabbrüche bei den Basenpaaren der Nukleinsäuren oder eine Reaktion innerhalb bzw. mit den Proteinen selbst erfolgt. Der Effekt durch Kraftfelder ist je nach Strom-Stärke kleiner oder grösser.

Der Erfindung liegt die **Aufgabe** zu Grunde, ein Verfahren bereitzustellen, das eine selektive Abreicherung, Inaktivierung, Stimulierung oder Lyse von (resistenten) biologischen Partikeln oder Zellen, wie z.B. Viren oder Bakterien, in unterschiedlichen Medien ermöglicht.
Vorzugsweise soll die Abreicherung, Inaktivierung, Stimulierung oder Lyse in biologischen, physiologischen und industriellen Flüssigkeiten möglich werden.
Eine weitere Aufgabe ist es, ein selektives Abreicherung-, Inaktivierungs- oder Lyse-Verfahren bereitzustellen, welches eine unerwünschte Komponente, z.B. die biologischen Partikel oder Zellen, aus der Flüssigkeit entfernen kann, wobei unerwünschte Effekte auf andere Bestandteile der Flüssigkeit, z.B. andere Zellen unterbleiben. Auch eine Änderung der Matrix (des Mediums) des pH-Wertes oder eine pH-Drift tritt nicht auf.
Die Inaktivierung von HIV -1 in Voll-Blut sowie die Inaktivierung resistenter Keime durch diese relativ einfache Technologie, wäre ein Durchbruch bei der Bekämpfung und Eindämmung von Krankheiten, die auf virale und bakterielle Infektionen zurück zu führen sind.

Die **Lösung der Aufgabe** geschieht durch die gezielte Kombination unterschiedlicher, physikalischer Effekte (Elektrolyse, Elektroporation, Elektrophorese, Voltage Depending Gating,Oszillation mit einer variablen Pulsweiten- und Frequenz-Modulation- nacheinander und nebeneinander bzw. überlagernd - zur gleichen Zeit - im Gleichspannungs-Nieder-Voltbereich. Dadurch können Zustände einer angeregten oder letalen Oszillation / Resonanz bei biologischen Partikeln erreicht werden. Die Pulsung geschieht also nicht um thermische Effekte auszulösen, die Polaritätsänderung bei der Elektrolyse zu umgehen, sondern um beispielsweise die elektrolytischen Reaktionen welche elektrodenbedingt ausgelöst werden können, einzuschränken. Für das Gelingen ist die Kontrolle / Anpassung aller apparativen und physikalischen Parameter (die später im Detail genannt werden) unabdingbar.

Weiterhin ist eine bevorzugte Anwendung die Stimulierung einer Zelle oder eines biologischen Partikels durch eine Oszillation, durch Pulsung unter Elektrolyse-Bedingung, wobei ein permeabler Zell-Zustand erreicht wird, der es erleichtert, chemische Komponenten die durch Elektrolyse dargestellt wurden, zur Reaktion, zu bringen oder auch Gene (Transfektion bei eukaryotischen Zellen), Moleküle (Wirkstoffe) einzuschleusen und eine Funktion als Übertragungs-System, zu ermöglichen. Man spricht hier von "Ghost Cells" als "Biological Carrier-System" verschiedenen Ursprungs z.B. Human-Zellen, tierische Zellen oder Bakterien-Zellen.

Eine Differenzierung in biologischen Flüssigkeiten gelingt aufgrund der Tatsache, dass der Zellaufbau von Eucyten und Protocyten signifikant unterschiedlich ist. Mikroorganismen besitzen eine Zellwand und eine Zellmembrane - daraus folgend ist eine Stimulierung durch Pulse (Oszillation) bei unterschiedlicher Pulsfrequenz und Pulsenergie möglich - beide Parameter sind für eine Zellart (Eucyten / Protocyten) unterschiedlich. Diese Effekte sind auch bei dem Vorgang der reinen Elektrolyse feststellbar, da bei hohen Spannungen und Stromstärken die Zelle irreversibel geschädigt wird. Diese Unterschiede wurden schon bei gramnegativen und grampositiven Keimen beobachtet, da unter gleichen Elektrolyse-Bedingungen (Osmolarität des Mediums, Temperatur, Spannung und Stromstärke) zuerst gramnegative Keime lysiert werden.

Eine flüssige, wässrige Matrix erleichtert dieses, da die Energie-Pulse ein optimales Übertragungs-Medium (Kopplungsmedium) vorfinden. Auch gerade die Form und Anordnung der Elektroden-Platten parallel zueinander zeigt die Eigenschaften eines Plattenkondensators, welcher prädestiniert ist zur Übertragung von Schwingungen auf das flüssige Medium und darin "gelöste", dispergierte oder emulgierte biologische Partikel.
Ein Vergleich bei gleichem Medium und biologischen Partikeln in Bezug auf die "Kill-Kinetik" bringt die nötigen Erkenntnisse zur Einstellung von Parametern wie die Strömungsgeschwindigkeit (Volumen / Zeiteinheit), Spannung und Stromstärke, Zellkonstante (Abstand der Elektroden im Verhältnis zur Fläche), Gesamtzeit der Elektrolyse bzw. Behandlung, pH-Wert der Flüssigkeit, Dichte, Leitfähigkeit, Temperatur usw.

Das erfindungsgemässe Verfahren weist den Vorteil der gezielten Kombination unterschiedlicher Techniken :
- Elektrolyse-Effekte bei Einsatz von Gleichstrom im Niedervolt-Bereich mit Umschaltung der Polarität im Sekunden- bis Minuten-Bereich;
- Elektrolyse-Effekte durch Pulsung der Gleichspannung im Niedervoltbereich ohne Polaritätsänderung;
- Frequenz- und Pulsweiten-Modulation von Hz bis KHz bzw. MHz unter Umgehung bzw. Reduktion der Elektrolyse-Effekte bis zu einer letalen Oszillation für eine Zelle bzw. biologischen Partikel;
in einem System auf.
Das spannungsabhängige Durchlassen (Voltage Depending Gating) und die Elektroporation sind in diesem System aufgrund der technischen Konfiguration als Übergangs-Zustand ebenfalls enthalten. Bei hoher Leitfähigkeit und Anwendung von höheren Stromstärken bzw. Spannungen sind ausserdem elektrophoretische Effekte sichtbar.
Das bedeutet, es handelt sich in keinem Fall um eine reine Anwendung von Elektrolyse, Oszillation, Elektroporation u.a., da im Gegenteil die Effekte gezielt unterdrückt oder gefördert werden.
Die Zeitintervalle können frei gewählt werden wo alle beschriebenen Modi aktiv sind, wobei eine Funktion primär vorherrschen kann oder zwei paritätisch oder gar drei bzw. vier Effekte entweder nacheinander oder überlagernd bzw. parallel anzutreffen sind.

Somit ist das erfindungsgemässe Verfahren mit einer technischen Grundausstattung bei der Verwendung von "Dimensions Stabilen Anoden" (DSA) - also einer Elektrode die keine lonen an das Medium abgibt - wesentlich flexibler und kann an die Applikation bzw. an das Flüssigkeits-System angepasst werden.
Die Elektroden können als metallisches Substrat z.B. Ti, W, Zr, Nb, Ta und andere Metalle einsetzen. Für leitfähige, keramische Substrate können Kohlenstoff-Allotrope oder Silikon eingesetzt werden. Generell sind alle Metalle / Nichtmetalle oder MischOxide in verschiedenen Kombinationen für Elektroden denkbar.

Für die Funktion zu Erzeugung einer letalen Oszillation wird ein Frequenz-Generator von 10 Hz bis 1 MHz realisiert, der gleichzeitig die Möglichkeit hat die ausgegebene Frequenz auch im Tastverhältnis, zu ändern (Pulsweiten-Modulation). Der so erzeugte "Frequenz-Burst" wird abhängig von den Umschalt-Intervallen über Leistungsstufen auf die planparallelen Elektroden geschaltet. Der Abruf der Energie geschieht über ein geeignetes Spannungs-Netzteil (Power-Supply) und die Pulsweiten- und Frequenz-Modulation über eine elektronische Schaltung mit Mikroprozessoren, die eine Regelung für beliebige Applikationen erlauben. Hier kann z.B. sichtbar über ein Display die Amplitude, die Frequenz und das Tastverhältnis abgelesen werden, um für jedes System optimale Parameter auszuwählen, welches weiterhin vorteilhaft zur Umsetzung für neue Anwendungen ist. Keine der bekannten, publizierten Verfahren oder Methoden nutzt zielgerichtet und optimiert die beschriebenen Modi zusammen in einem System.

zur Inaktivierung / Lyse von resistenten Keimen, wie z.B. Staph. aureus, E. coli, Myocobacterium tubercolosis u.a. sowie einer Inaktivierung von Viren bzw. Phagen (z.B. MS2-Phagen, HIV, Hepatitis B usw.) in Nährstoff-Lösungen oder anderen physiologischen Flüssigkeiten, wird die Fluss-Elektrolyse - nicht thermisch - durch Anlegen einer Gleichspannung im Niedervolt-Bereich an eine Elektrolyse-Zelle im mV- bis V-Bereich ohne Diaphragma, nicht pulsierend, mit einer Polaritätsumschaltung im Sekunden- bis Minuten-Bereich eingesetzt.

Durch die chemische Reaktion der Oxidation oder Halogenierung reagieren Nuklein-Säuren wobei die Ketten zerstört werden (Kettenabbruch) oder andere funktionellen Gruppen wie z. B. in den Membranproteinen , so dass Zellmaterial irreversibel geschädigt wird. Oxidation durch beispielsweise Ozon entfernt sicher Bakteriophagen, die beispielsweise bei Fermentations-Prozessen als Phagen-Infektion grosse Probleme bereiten. Bei Mikroorganismen wird durch Ozon die Zellmembran geschädigt. Dieses führt zu einer erhöhten Permeabilität. Die Membran verliert die Eigenschaft einer physiologischen Barriere. Membranproteine können direkt durch Ozon oder durch den radikalischen Mechanismus (Hydroxyl-Radikal-Reaktion, OH*) oxidiert werden. Durch schon sehr geringe Mengen an O₃ / OH* entstehen Fehler in Basenpaaren der DNS. Höhere Ozon- / OH* - Dosen bewirken unspezifische Kettenabbrüche an den Einzel- oder Doppelsträngen der Polynucleotidketten.
Die physikalische Effekte sind gekennzeichnet durch ein hohes Redoxpotenzial, stärkere elektrische- und magnetische Felder mit hoher Dichte der Stromstärke bei mehr als 20 bis 200 mA / cm² und einer Ladungsmenge von mehr als 5 bis 200 Coulomb pro Liter Flüssigkeit.

### Beispiele:

Die bereits beschriebenen Bedingungen gestatten es, eine kontinuierliche, selektive Abreicherung, Inaktivierung und Lyse von Bakterien, Hefen / Pilzen, Viren und Phagen in Vollblut durch zu führen.
Dieses gelingt auch in Medien die physikalisch, physiologisch und chemisch ähnlich sind wie z.B. PBS-Medium, TSB-Medium oder MRD-Medium. Kennzeichen dieser Medien ist eine sehr hohe Leitfähigkeit im Bereich von 1 bis 50 mS/ cm bei Raumtemperatur.
Eine Inaktivierung von Hepatitis B Viren (HBV) und HIV gelingt schon durch einfaches, elektrolytisch, oxidiertes Wasser durch Elektrolyse einer 0,05%-igen Kochsalz-Lösung innerhalb von 45 Minuten bei etwa 12 Volt und 3 Ampere. Hierbei betrug das Redoxpotenzial 1053 mV und die (freie) Gesamt-Rest-Chlor-Konzentration betrug 4,20 ppm.

Die Lipidhülle bei behüllten Viren (HIV, Hepatitis B,C oder D oder auch Herpes) ist bei kleineren Salzkonzentrationen und bei geringeren Stromstärken besser zugänglich um eine Inaktivierung, zu erreichen. Unbehüllte (harte) Viren sind hingegen recht stabil und erfordern hohe Salzkonzentrationen und hohe Stromstärken bei einer mit Metalloxiden aktivierten Titan-Anode. Nur durch Addition einer starken Oszillation im Bereich von 1500 bis 2000 Hz bei einer Amplitude von 3 bis 15 Volt werden harte Viren inaktiviert.

Während bei MS2-Phagen unter katalytischer Elektrolyse in halbverdünnten PBS-Medium auch bei normalen Stromstärken von etwa 0,9 bis 1,1 A und einem Fluss von 30 1/h kaum eine signifikante Abreicherung gelang (etwa eine Zehnerpotenz) ohne Oszillation gelingt.
Der Einsatz von ozonbildenden Elektroden bei der Fermentation hingegen lies bei einer Phagen-Infektion einer Lactobacillus spp. Kultur (Herstellung von Apfelwein - Cider) die Phagen-Infektion vollständig bereinigen. Dieser Versuch fand nur unter Elektrolyse-Bedingungen 3,8 Volt Gleichstrom ungepulst bei 0,6 Ampere bei hohem Durchfluss von mehr als 60 Liter / Stunde mit einer platinierten Titan-Anode statt.

Wie aus verschiedenen Versuchen zu entnehmen war, stellt Voll-Blut eine echte Herausforderung dar. Zwei Parameter, A. zellstimulierende Effekte und B. die Zytotoxizität für die primären Leukozyten, stehen hierbei im Vordergrund der zu untersuchenden Nebeneffekte. Dieses ist relativ einfach bestimmbar denn im nativen Blut sind im Normalzustand nur etwa 3% der Leukozyten aktiviert. 2000 ml frisches Pferdeblut wurden mit 200 ml einer 3%-igen (gesättigt) Di-Natrium-Oxalat-Lösung (als Vorlage) versetzt, um eine Koagulation zu verhindern. Diese Probe wurde mit Pseudomonas putida etwa 10⁶ / ml versetzt (gespikt). Es wurde unter Rühren mit einem Magnetrührer bei 25°C und einem Fluss von 30 Liter pro Stunde bei 3,0 Volt und 0,4 Ampere der dynamischen, katalytischen "Oszillation-Elektrolyse" unterworfen. Es wurde in Abständen von je 2 Minuten Proben genommen und ausplattiert - wobei nach 6 Minuten kein Wachstum mehr registriert werden konnte.

Blut ist eine gefärbte Körperflüssigkeit mit grob 55% Blutplasma and 45% Zellen-Anteil. Die drei Hauptzelltypen sind Erythrozyten, Leukozyten und Thrombozyten. 92% des Blutplasmas besteht aus Wasser und die restlichen 8% enthalten Proteine, Metaboliten und lonen. Blutplasma und der gesamte Inhalt wird Voll-Blut genannt. Die Dichte von Blut (human) ist ungefähr 1,060 g/ml.
Der pH-Wert liegt bei etwa 7,41 und das durchschnittliche Volumen eines normalen Menschen (bei etwa 70 Kg) beträgt 5-6 Liter.
Die Leitfähigkeit liegt bei 40 bis 50 mS / cm bei Raumtemperatur und die Osmolarität ist vergleichbar mit Meerwasser ungefähr 300 mosm / 1.
Die Viskosität liegt im Bereich von 2,30 bis 2,72 × 10⁻³ Pa × s bei 37°C.
- Dieses entspricht fast "idealen" Bedingungen für die Durchführung einer Elektrolyse. Leider wird aber unter diesen Bedingungen der Elektrolyse mit Nebenwirkungen bei andere Zellen, zu rechnen sein, so dass diese Applikation ungeeignet ist, da die Wechselwirkungen auf andere biologische Partikel einfach zu gross ist.

Generell kann man mit der gleichen Vorrichtung - aber unterschiedlichen Elektrodenmaterialien, sowie unterschiedlichen physikalischen Parametern eine Dekontamination des Blutes von resistenten Bakterien oder Viren erreichen.
Für den Fall der Viren- bzw. Phagen-Inaktivierung in wässrigen Systemen setzt man am besten eine Diamant-Elektrode (auf Niob-Basis) oder eine platinierte Titan-Anode ein. Das Arbeitspotenzial der genannten Elektroden ermöglicht die Darstellung von Ozon oder OH*-Radikalen bei auch kleineren Stromstärken und konzentrierten Elektrolyten (1-50 mS / cm bei 20°C). Allerdings sind durch die Hydroxyl-Radikale Nebenreaktionen zu befürchten.
Ein einfaches Modell, um die optimale Stromstärkendichte in Bezug zum COD - Verbrauch (Carbon Oxygen Demand) zu erhalten, wurde von P.A. Michaud präsentiert.
1 (mA/cm²) = 0.024 COD (mg O₂/l)
Die Elektroden-Oberfläche in cm² steht hierbei in direktem Verhältnis zur angewendeten Stromdichte (mA/cm2) über die Zeit. Durch die richtige Wahl des Elektrodenabstandes z.B. 1 mm (Zellkonstante), des Flusses über die Zeit, des Gehaltes an Elektrolyt oder besser Chloridkonzentration, können Stromdichten von 150 mA/cm² erhalten werden, wobei die Chlorid-Konzentration bei etwa 80 ppm lag.
Bei einer Kontaktzeit von etwa 5-10 Minuten werden 99,99% einer Legionellen Kontamination (ewa 10⁴ Keime / ml) mit der oben bezeichneten "Diamant-Elektrode" inaktiviert.

Da OH*-Radikale sehr reaktiv sind, treten unspezifische Nebenreaktionen auf. Bei kleineren Spannungen unter 3 Volt ist die Inaktivierung von Viren (HIV) sichtbarsie nimmt aber bei noch kleineren Werten ab, da die Produktion von Ozon oder auch Hydroxyl-Radikalen mit geringerer Stromstärke abnimmt. Die Stimulierung aller Zellen insbesondere der Leukozyten oder aber unspezifische Nebenreaktionen sind unerwünscht.
Zur Vermeidung von Radikalbildung wird die Spannung abgesenkt auf Werte von 0,5 bis 3 Volt bei einer Stromstärke von 0,1 bis 0,3 Ampere und die Gleichspannung im Bereich von 100 bis 2000 Hz gepulst.
Die Lösung ist eine Balance zwischen sehr kleinem Elektrolyse-Effekt und einer Pulsation bzw. letalen Oszillation, um den Anteil an Nebenreaktionen möglichst niedrig zu halten oder auszuschliessen. Hier liegt der Fluss des Mediums durch die Zelle bei 10 bis 50 1/ Stunde. Die Grösse der aktiven Elektroden Oberfläche beträgt in diesem Fluss-Bereich 200 bis 220 cm² und der Elektroden-Plattenabstand 1 bis 5 mm.

Während eine Metalloxid-aktivierte Titan-Anode (Ti, Ru, Ir - TiO₂ oder Ti,Ru,lr,ln oder Ti,Ru,lr,Co) wie in der Literatur beschrieben, ausreicht um eine Abreicherung und Lyse von resistenten Bakterien (z.B. MRSA), zu erreichen gelingt eine Inaktivierung von "unbehüllten" (harten)Viren / Phagen nur mit platinierten Titan-Anoden (Beschichtung von 1 bis 3 µm Platinschicht) bzw. Diamant auf Niob beschichteten Elektroden in industriellen- und physiologischen Flüssigkeiten bei primär Elektrolyse-Effekten.
Eisen-Ionen oder auch andere lonen wie Kupfer, Zink (primär mach der Spannungsreihe unedle Metalle) können eine Reaktion als Niederschlag mit Blut (biologische Flüssigkeit) ergeben und werden deshalb für diese Matrix in der Anwendung ausgeschlossen.

Es ist möglich, eine Kombination der Elektroden oder eine in Reihe-Schaltung von zwei Zellen für beide Applikationen anzustreben. Dieses ist generell möglich erfordert aber zwei vollkommen, getrennte Stromversorgungen bzw. Regelungen da die physikalischen Parameter für Viren / Phagen bzw. resistente Bakterien zu unterschiedlich sind. Bei biologischen Flüssigkeiten ist aber aufgrund der zu erwartenden Nebenreaktionen äußerste Vorsicht geboten.
Dieses bedeutet eine Hardware für die zwei unterschiedlichen Applikationen mit allerdings unterschiedlichen Elektroden-Materialien (Anoden) und unterschiedlichen Einstellungen der physikalischen Parameter.

Um in Praxis erfolgreich vorzugehen und eine kontinuierliche, selektive Inaktivierung "biologischer Zellmasse", zu erreichen, wurden in einem Kurzversuch isoliert Bakterien, Hefen oder Phagen in einem definierten Medium (PBS) der Elektrolyse unterworfen.
Hierbei wird zuerst mit kleinen Spannungen gestartet z.B. 0,5 bis 1,0 Volt, dann in Schritten gesteigert auf 3,0 und 4,0 usw. (oder in noch kleineren Intervallen z.B. 0,1 V) wobei sich entsprechende Stromstärken je nach Leitfähigkeit des Mediums ergeben.

Hierbei ergibt sich, vorteilhaft mit einem Analysengerät z.B. Partikel-Counter, welches online in Echtzeit betrieben wird, eine Abreicherungskurve. Konzentrationen von Bakterien oder Hefen im Bereich von 10e8 als Startkonzentration können so bis auf einen Level von 10e4 bei klarem Hintergrund gut sichtbar in Echtzeit abgereichert werden. Die Abreicherung läuft natürlich bis auf 0 Keime herunter ist aber als Nachweisgrenze in Echtzeit nicht sichtbar bis in diesen Bereich. Die Kinetik der erhaltenen Kurve gibt Aufschluss über die Effektivität der Bedingungen. Andere klassische Verfahren zum Erhalt des Resultats können ebenso eingesetzt werden, haben aber den Nachteil, dass es nicht in Echtzeit - also sofort vorliegt.

Der Mechanismus einer Inaktivierung bei Bakterien ist unterschiedlich, da eine Zelle metabolisch inaktiv werden kann, ab er die Zellgestalt noch erhalten bleiben kann. Dieses ist im Partikel-Counter bedingt sichtbar und kann nur mit Hilfe eines hochauflösenden Mikroskops erfasst werden oder mit dem Flow-Cytometer.
Man fährt nun bei gleichem Fluss, pH-Wert, Salzkonzentration und Medium-Zusammensetzung entsprechende Spannungs- / Stromstärken-Kurven, wobei man im unteren Bereich z.B. bei 1 Volt anfängt und dieses beliebig steigert auf z.B. 7 Volt. Hierdurch erhält man eine Darstellung der "Kill-Kinetik" für ein bestimmtes System z.B. E. coli mit MS2-Phagen in PBS.

Je nach Leitfähigkeit und anliegender Stromstärke kann man ein bestimmtes Volumen mit entsprechender Biomasse, z.B. 10 Liter in einem Lauf, bei der Durchführung durch die Reaktions-Zelle abreichern. Eine Phagen-Infektion einer Bakterienkultur lässt sich auch online mit obiger Methode (Partikel-Counter) ableiten, da signifikant in erster Fraktion Zell-Debris auftritt (0,4 bis 0,7 micron).

Hat man nun ein bestimmtes Protein aus einer Bakterien-Kultur zu isolieren, verfährt man ähnlich wie oben beschrieben, nur mit dem Unterschied, dass die Parameter der Elektrolyse im Medium zusätzlich auf den Erhalt der biologischen Aktivität abgestimmt sind. Bei hohen Biomasse-Konzentrationen ist ein Verfahren zu wählen, dass z.B. einen eingesetzten E. coli- Stamm nicht nur inaktiviert sondern die Zelle bevorzugt zum Platzen bringt - also eine echte Lyse herbeiführt. Hierbei kann es notwendig werden, die Bedingungen der Elektrolyse anzupassen auf so genannte "milde Bedingungen" beispielsweise bei höherem Fluss (l/h) und geringerer, minimaler Strom-Stärke, um sicherzustellen, dass die biologische Aktivität des Proteins beibehalten wird. Wenn dieses nicht in einem Durchlauf durch die Elektrolyse-Zelle erreichbar ist, kann dieses wiederholt durchgeführt werden. Der Einschluss-Körper (inclusion body) konnte mit dem Partikel-Counter erfasst werden (Protein-Aggregat), ebenso wie die Abreicherungs-Kinetik. Man kann sogar unterschiedliche "Inclusion Bodies" differenzieren - bei 0,95 micron für E. coli mit Protein und bei 1,25 micron für E. coli mit Plasmid-DNA.

Bei hoher Konzentration der Biomasse ist eine "Zehrung" nur unter den Bedingungen der Elektrolyse sichtbar. Es empfiehlt sich ausserdem ein Umpumpen vom Fermenter in einen Vorratsbehälter und keine zyklische Arbeitsweise. Der Vorteil dieses Verfahrens ist aber der, dass man die unter [045] beschriebenen Parameter (11 verschiedene Parameter) auswählen kann und auch Kombinationen zur Lösung heranziehen kann. Damit ist dieses vorgeschlagene Verfahren viel besser justierbar (fine tuning) und erfolgversprechender.

Bei der Darstellung (Freisetzung als disruption method) von rekombinanten Proteinen, Plasmid-DNA oder Oligo- bzw. Polysaccharide nimmt diese neue vorgeschlagene Methode eine Schlüsselstellung ein, da sie auch skalierbar ist.

Nimmt man also ein fixes Volumen z.B. 10 Liter Nährlösung und pumpt ein Medium wie oben beschrieben durch eine Elektrolyse Zelle unter Normalbedingung ergeben sich Abreicherungsfraktionen.
Die Abreicherung kann durch folgende Einflüsse (Parameter) gesteuert werden, die einzeln oder in allen möglichen Kombinationen variiert werden können:
- Fluss d.h. Liter Medium pro Zeiteinheit
- Durch die angewendete Spannung bzw. daraus resultierende Strom-Stärke
- Durch die verfügbare, aktive Austauschfläche der Elektrolyse-Elektroden
- Durch das eingesetzte Elektroden-Material und dessen Affinität zu Chlor-oder Sauerstoffproduktion oder Ozon - / Hydroxyl-Radikal-Produktion z.B. Ti als Träger mit Ru-, Ir- und In-Oxiden oder platinierte Titan-Anoden oder Diamant auf Niob-Anoden
- Durch den Elektrodenabstand und vorzugsweise planparallele Anordnung (Kondensatorprinzip)
- Durch die elektrische Feldstärke
- Durch die gewählte Zeitkonstante bei der Polaritätsumschaltung im ungepulsten DC-Mode
- Durch das Verhältnis des Partialdruckes von Wasserstoff zu Sauerstoff (PH₂ / PO₂) oder auch Ozon im Medium - vorzugsweise Lösung.
- Durch den pH-Wert und Temperatur sowie vorzugsweise den ChloridGehalt oder anderen Anionen oder beliebige Kationen, wobei auch eine Pufferung des Systems (Medium / Lösung) Einfluss hat
- Zeit / Dauer der Elektrolyse
- Durch die Pulsdauer, Pulsabstand, Pulsform und Amplitude
- Durch den erzeugten "Burst" als Oszillation mit gleichzeitger Elektrolyse
Es wird angenommen, dass für ein bestimmtes Medium unter Normalbedingung für bestimmte Keime oder Viren eine definierte, letale Oszillation / Resonanz, zu finden ist.

Durch das vorgeschlagene Verfahren zur Behandlung von Nährstofflösungen (Nährmedien), Salzlösungen usw. kann "selektiv" von Mikroorganismen oder Viren /Phagen befreit werden. Hierbei kann in einem PBS-Medium unter Normalbedingung folgende Reihenfolge (Fraktionierung) erwartet werden:
A Innerhalb von Sekunden werden Bakterien abgetötet (zuerst gram-negative) B Innerhalb von Minuten bei gleicher Stromstärke Hefen / Pilze
C Innerhalb von Minuten / Stunden bei etwas erhöhter Stromstärke sehr "harte" Phagen (MS2-Phagen) bzw. nicht umhüllte Viren z.B. Poliovirus.
   Dieses gilt bei Metalloxid aktivierten Titan-Anoden (MOX-Ti-Anoden)
D Bei Einsatz von platinierten Titan-Anoden, oder Diamant auf Niob erreicht man eine Inaktivierungen von Phagen im gleichen Medium (PBS) innerhalb von Minuten.

Man kann die Selektion nach den oben [045] genannten Parametern steuern. Ziel kann es sein eine Mischung aus Bakterien, Hefen (Pilzen) und Phagen selektiv in Fraktionen, zu lysieren bzw. abzureichern. Generell kann man feststellen, dass gramnegative Keime schneller der Abreicherung / Lyse durch die katalytische Elektrolyse (ohne Pulsung) unterliegen als grampositive Keime.
Eine Probe von TSB wurde einer Behandlung - mit Unterdrückung der Elektrolyseunterzogen (Edelstahl - V4A- Elektrode / Ti-MOX-Kathode) unter Normalbedingung, bei einem Fluss von 60-70 ml / Minute, bei 130 Hz (Monopulse / positiv) mit etwa 0.5 joule / ml - mit einem Ergebnis der Abreicherung von 10e5 Keimen / ml für E. Coli.

Eine weitere aber andere Applikation zur selektiven Abreicherung, Inaktivierung und Lyse gelang bei Milchserum welches enzymatisch aus Rohmilch hergestellt wurde und bei Rohmilch selbst. Bei einem Fluss von 25 I / Stunde (unter Normalbedingung) wurde mit einer Titan-MOX-Anode schon bei 0,2 A und 0,5 V eine Abreicherung von 1,6 x 10e6 Keimen / ml auf etwa 18.000 Keime erreicht - vorzugsweise erst gramnegative Keime. Es herrschten hier vorwiegend elektrolytische Bedingungen. Bei Rohmilch selbst, wurden primär erst somatische Zellen lysiert was bei der Grösse der Reaktions-Zelle (aktive Reaktionsfläche) und Zellkonstante keine Abreicherung von Bakterien zuliess. Erst als alle somatischen Zellen eliminiert waren trat signifikant eine Abreicherung von Bakterien ein.
Unter Erhöhung der Stromstärke auf 5,0 A bei etwa 5,5 V konnte eine selektive Entfernung von Protein durch Ausfällen erreicht werden, welches isoliert wurde.

Eine Selektion bei biologischen Flüssigkeiten (Blut) bei der Entfernung von Bakterien, ohne andere Zellen zu beeinflussen, gelingt bei der Elektrolyse ohne Pulsung mit Gleichstrom kaum und nur bei kleinen Stromstärken. Behüllte Viren werden unter ähnlichen bzw. gleichen Bedingungen entfernt. Unbehüllte Viren und einige Phagen sind unter diesen "milden" Bedingungen der Elektrolyse nicht zugänglich ohne andere Zellen signifikant, zu beeinflussen. Sie sind nur durch eine letale Oszillation / Resonanz in Verbindung mit der Elektrolyse zugänglich. Generell ist die Elektroyse allein (extrakorporale) von Voll-Blut ungeeignet, wegen der zu hohen Risiken von Nebenwirkungen (Zellschädigungen).

Die Vorteile durch die Lösung der vorgeschlagenen Methode sind zahlreich. Eine Anpassung aller Parameter an unterschiedliche Medien bei Darstellung von biotechnologischen Produkten (z.B. Proteine oder Plasmid-DNA) ist erforderlich, um jede Elektrode im Verhalten / Perfomance , zu überprüfen oder aber auch um die Effektivität der Geschwindigkeit der Inaktivierung, festzulegen. Diese Anpassung ist, aber aufgrund der vielen, zur Verfügung stehenden Parameter, ein besonderer Vorteil dieser vorgeschlagenen Methode.

Das erfindungsgemässe Verfahren weist weiterhin den Vorteil auf, dass die Betriebskosten für einen m³ Voll-Blut bei etwa 60 W h Energie-Verbrauch liegen, sonst aber keine weiteren Kosten (ausser beim Temperieren der Apparatur) im Betrieb anfallen.

Durch die vorgeschlagene Methode könnte erstmals ein Stadium in der Medizin erreicht werden, dass aufgrund einer physikalischen Methode eine Elimination von HIV-1 erfolgen könnte, da bei wiederholter Anwendung an einer HIVpositiven mit Viren infizierten Person ein klinischer Zustand erreicht werden könnte, der frei von HIV-1 lauten könnte.
Das erfindungsgemässe Verfahren gleicht weiterhin bei der Abreicherung resistenter Keime im Blut den Mangel an effektiven Antibiotika aus, da die physikalische Methode keine (neue) Resistenzbildung zulässt, aber resistente Keime inaktiviert. Der volkswirtschaftliche Vorteil dieser Methode ist unübersehbar. Bei dem biotechnologischen Einsatz der erfindungsgemässen Vorrichtung und Verfahren können verfahrenstechnisch grössere Volumina von 10 bis 50 m³ Fermentations-Medium beim Aufschluss der "Protein-Einschluss-Körper" (inclusion bodies) unter Beibehaltung der biologisch, aktiven Form - isoliert werden (disruption method).

Nachgeschaltete Verfahren zur Aufreinigung von Proteinen erfahren eine wesentliche Erleichterung da die Detergenzien entfallen, die bei chromatographischen Aufreinigungsschritten den Iso-elektrischen Punkt verschieben können, eingesetzt werden, nun entfallen und damit eine bessere Aufreinigung möglich ist. Dieses führt zu höheren Ausbeuten und biologisch aktiveren Molekülen. Andere mechanische Zellaufschluss-Methoden wie z.B. "French-Press" sind für technische Anwendungen ungeeignet da die die kleine "Pore" strömungsbedingt nur kleine Volumina über die Zeit zulässt.

Im folgenden werden Ausführungsformen der vorliegenden Erfindung unter Bezugnahme auf die beigefügte Zeichnung beschrieben. In der Zeichnung zeigt:

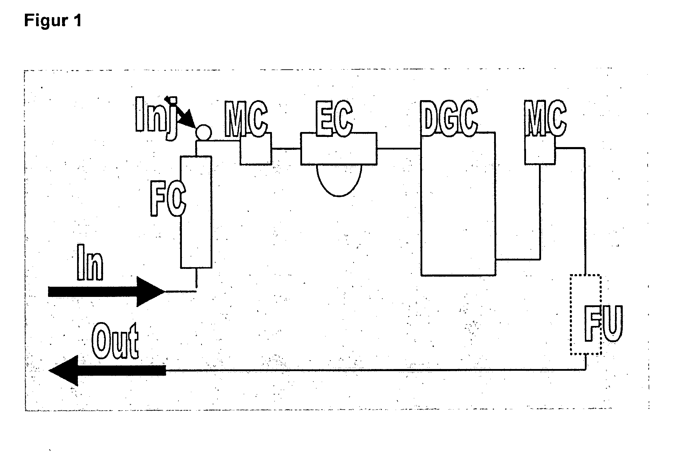

- Figur 1: eine Ausführungsform der Vorrichtung die einzelne Komponenten des Systems zeigt - für die Anwendung von Voll-Blut.
**Figur 1** zeigt eine Vorrichtung zur Inaktivierung resistenter Keime und Viren in flüssiger Matrix (Voll-Blut), gemäss einer Ausführungsform der vorliegenden Erfindung.
Durch (**In /1**) gekennzeichnete Stelle bezeichnet die Blutabnahme (iV) eines Patienten, bei der die Weiterleitung durch z.B. geeignete Schlauchverbindung in den **(FC / 2)** Flow-Controller (Fliessgeschwindigkeit / Pump-Geschwindigkeit). Der Flow-Controller regelt über einen Mikroprozessor die Pump-Geschwindigkeit, wobei verschiedene Ausführungsformen denkbar sind. (**Inj /3**) dosiert eine konstante Menge an Anti-Koagulantien zur Vermeidung der Blutgerinnung. Das Voll-Blut wird nach dem Injektor in eine dynamische Mischkammer **(MC /4)** geleitet um eine Sedimentation durch Mixen / Rühren auszuschliessen um dann in die Reaktions-Zelle (**EC /5)** zu gelangen, die temperierbar sein kann.
Nach Verlassen der Reaktions-Zelle (EC) wo die Inaktivierung von resistenten Keimen / Viren geschieht wird das Blut (Flüssigkeit) in eine Entgasungs-Einheit **(DGC / 6**) geleitet, die temperierbar ist. Diese Entgasungs-Einheit befreit von minimalen Restgasen (Wasserstoff, Sauerstoff, Chlor oder Ozon je nach Elektrode) die sich bei der Dekontamination bilden könnnten. Im einfachsten Fall besteht diese aus einem Kunststoff-Schlauch (Coil) von einigen Metern der für Gase durchlässig ist und im Vakuum betrieben wird. Im Anschluss nach Entgasung wird das Medium (Blut) wieder einer Mischkammer**(MC** /**7)** zugeführt, um eine Sedimentation zu vermeiden. (**FU** / **8)** ist eine optionale Filtrationseinheit mit einer optionalen Luftblasenfalle. Diese Filtrationseinheit kann aus synthetischen oder Zellulose-Material bestehen und könnte in "Cross-Flow-Verfahren" Zell-Debris entfernen. Mit gleicher Einheit könnte aber auch durch Adsorption z.B. über Nano-Material, eventuell freigesetzte Toxine (Jarrisch-Herxheimer Reaktion) gebunden werden. Danach wird unter Kennzeichnung **(Out / 9)** dem Patienten (iV) das Blut wieder zurückgegeben.

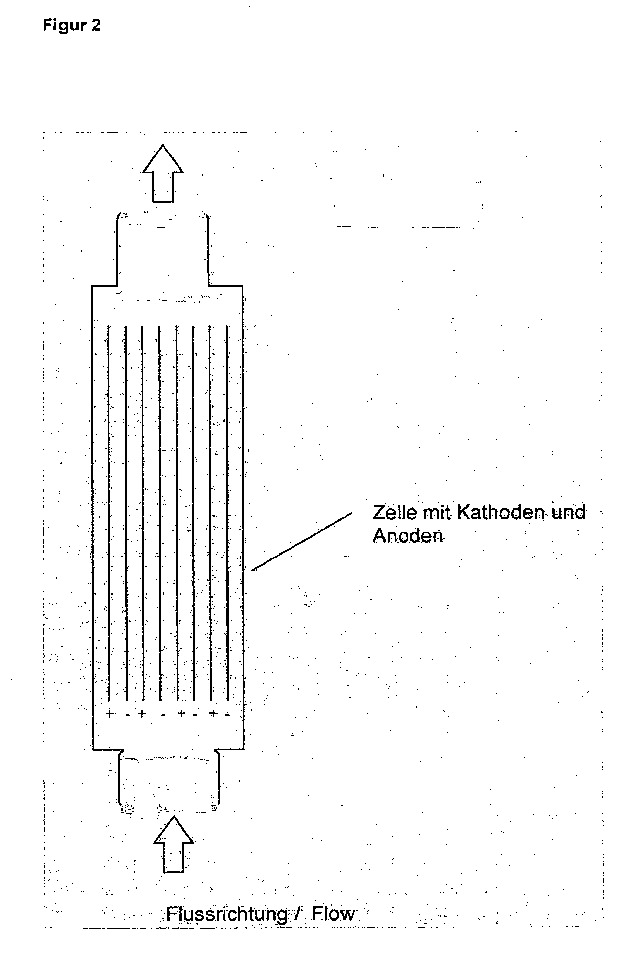

**Figur 2** zeigt eine Ausführungsform, Teil einer Gesamt-Vorrichtung, einer Reaktions-Zelle (EC 5 - in Figur 1).

| **Figur 1** | | |
|---|---|---|
| **In** | **1** | **Blut-Abnahmestelle zur Weiterleitung in die Vorrichtung** |
| **FC** | **2** | **Flussregelung (Flow-Controller) z.B. 3,0 Liter / Stunde** |
| **Inj** | **3** | **Injektor für z.B. Anti-Koagulantien** |
| **MC** | **4** | **Mischkammer (Mixing-Chamber)** |
| **EC** | **5** | **Reaktions-Zelle (temperierbar)** |
| **DGC** | **6** | **Entgasungseinheit (Degassing Chamber) temperierbar** |
| **MC** | **7** | **Mischkammer (Mixing-Chamber) - optional temperierbar** |
| **FU** | **8** | **optionale Filter-Einheit - optional mit Luftblasenfalle** |
| **Out** | **9** | **Ausgang aus der Vorrichtung - Rückführung zum Patienten** |

## Patentansprüche

1. Verfahren zur selektiven Abreicherung, Inaktivierung, Elimination, Stimulierung oder Lyse von biologischen Partikeln - in-vitro / in-vivo - in biologischen- , physiologischen- und industriellen Flüssigkeiten durch die gezielte Behandlung mit unterschiedlichen physikalischen Effekten die sich überlagern, parallel ablaufen und /oder nacheinander geschaltet werden - in allen Kombinationen - als dynamische Methode die skalierbar ist
**dadurch gekennzeichnet,**
**dass** eine Flüssigkeit mit einem definierten Fluss durch eine biokompatible Apparatur geleitet wird, die eine Behandlung durch gezielte, gleichzeitige, verschiedene physikalische Effekte ermöglicht, die sekundär chemische Folgeprodukte entstehen lassen kann, bei Leitfähigkeiten von Flüssigkeiten im Bereich von 0,01 µS / cm bis 1000 mS / cm bei beliebigen Temperaturen und beliebigem Druck.

2. Verfahren nach Anspruch 1
**dadurch gekennzeichnet,**
**dass**, eine Gleichspannung im Bereich von 0,1 Volt bis 100 Volt an Elektroden angelegt wird, die in abwechselnden Zeitintervallen (zeitlich gesteuert)
a.) im Pulsbetrieb oder ungepulst mit einer Oszillation und
b.) im normalen, ungepulstem Gleichstrom geschaltet werden oder
c.) wie bei a.) und b.) gleichzeitig, hintereinander oder überlagert ablaufen kann mit einer variablen Frequenz- und Pulsweiten-Modulation im Bereich von 10 Hz bis 1 MHz bei einer Amplitude von vorzugsweise 0,1 bis 20 Volt, bei allen Wellenformen (Sinus, Quadrat, Triangel usw.) vorzugsweise als Monopuls (positiv) - aber auch in allen anderen Pulsformen (bipuls / bipolar) erlaubt.

3. Verfahren nach Anspruch 1 oder 2
**dadurch gekennzeichnet,**
**dass** eine Transfektion von Zellen dynamisch (im Fluss) in skalierbarem Massstab erfolgen kann - also eine Einschleusung von heterologer DNA in Eukarontenzellen. Desgleichen eine Stimulation einer Zelle erfolgt (z.b. durch Effekte der Elektroporation) z.B von einer Säugerzelle erfolgt, wodurch eine "reversible" Öffnung z.B. ein Art Tunnel in der Membrane entsteht mit einem Durchmesser von beispielsweise 40 bis 200 nm, wodurch ein Teil des Zytoplasmas austreten kann aufgrund des hohen osmotischen Drucks in der Zelle und ein Molekül z.B. Arzneimittel oder aber genetisches Material in die Zelle eingeschleust wird, um eine "Geister-Zelle" (ghostcell) entstehen zu lassen für eine therapeutische Anwendung als biologisches Übertragungs-System (Biological Carrier System), die nach diesem Verfahren skalierbar ist, um im technischen Bereich (Produktion) eingesetzt, zu werden.

4. Verfahren nach Anspruch 1,2 oder 3
**dadurch gekennzeichnet,**
**dass** eine dynamische Zellaufschlussmethode (disruption method) die zur Lyse der Zelle führen kann, skalierbar für beliebige Volumina pro Zeiteinheit zur Freisetzung von rekombinanten Proteinen, Plasmid-DNA, Oligo- bzw. Polysaccharide, monoklonaler Antikörper oder anderer Moleküle bzw. Verbindungen gestattet, die bei der fermentativen Herstellung z.B. durch E. coli (protein expression) dargestellt werden.

5. Verfahren nach Anspruch 1 bis 4
**dadurch gekennzeichnet,**
**dass** im dynamischen Prozess - Durchflussbetrieb - skalierbar durch Herstellen von Ozon oder Hydroxyl-Radikal beliebige Phagen aus einer Flüssigkeit (z.B. Fermentations-Broth) eliminiert werden können.

6. Verfahren nach einem der Ansprüche 1 bis 5
**dadurch gekennzeichnet,**
**dass** im dynamischen Prozess, im Durchflussbetrieb - extrakorporal - Flüssigkeiten behandelt (z.B. Blut / Blut-Plasma) werden durch Stimulation bestimmter Zellen, um eine bessere oder schnellere Aufnahme von Wirkstoffen, zu ermöglichen als skalierbarer Prozess.

7. Verfahren nach einem der Ansprüche 1 bis 6
**dadurch gekennzeichnet,**
**dass** eine selektive Abreicherung von Biomasse im dynamischen Prozess (Durchfluss pro Zeiteinheit), skalierbar, z.B. in einem Fermenter erfolgt, wobei unterschiedliche Zellen bzw. biologische Partikel in Fraktionen entfernt werden können.

8. Verfahren nach einem der Ansprüche 1 bis 7
**dadurch gekennzeichnet,**
**dass** für biologische Flüssigkeiten, extrakorporal in einer biokompatiblen Apparatur, selektiv, resistente Bakterien oder Viren abgereichert bzw. inaktiviert werden im Durchflussbetrieb unter Umgehung von Schädigung anderer Zellen oder in der Flüssigkeit befindlicher anderer biologischer Partikel.

9. Verfahren nach einem der vorangegangenen Ansprüche
**dadurch gekennzeichnet,**
**dass** eine Anwendung (Zellaufschluss) im analytischem Bereich als Lyse-Methode (disruption method) zur Freisetzung von Zellinhaltsstoffen z.B. ATP unter Einsatz von multiplen Apparaturen / Vorrichtungen in Form eines biokompatiblen Strömungskörpers mit intergrierten Elektroden als "Einmal-Zelle" (disposable) eingesetzt wird, mit anschliessender Erfassung (Messung) dieser Parameter (z.B. mikrobielles ATP).

10. Verfahren nach einem der vorangegangenen Ansprüche
**dadurch gekennzeichnet,**
**dass** isotonische, hypotonische Lösungen oder auch Lösungen mit oder ohne Leitfähigkeit als auch Suspensionen und Emulsionen eingesetzt werden.

11. Verfahren nach einem der vorangegangenen Ansprüche
**dadurch gekennzeichnet,**
**dass** folgende Behandlung zusammen abläuft:
• Beeinflussung, Stimulierung biologischer Partikel durch elektrische-, magnetische und elektromagnetische Felder bzw. Feldstärken in Flüssigkeiten
• Ablauf von erzwungener Elektrolyse, Elektrophorese, Voltage Depending Gating, Elektroporation durch Anlegen einer äusseren Gleichspannung und Pulsung bzw. Nicht-Pulsung mit unterschiedlichen Zellkonstanten.
• Pulsation von Gleichspannung mit Frequenz- und Pulsweitenmodulation um biologische Partikel in Flüssigkeiten einer Oszillation / Resonanz auszusetzen, die zu verschieden physiologischen Zuständen von biologischen Partikeln führt.

12. Verfahren nach einem der vorangegangenen Ansprüche
**dadurch gekennzeichnet,**
**dass** Endotoxine (Lipopolysaccharide) zugehörig zur Gruppe der Pyrogene, die bei verschieden Verfahren oder bei der Lyse von Zellen auftreten können, inaktiviert werden durch partielle Oxidation.

13. Vorrichtung zur selektiven Abreicherung, Inaktivierung, Stimulierung, Elimination oder Lyse von biologischen Partikeln - in-vitro / in-vivo - in biologischen-, physiologischen- und industriellen Flüssigkeiten
**dadurch gekennzeichnet,**
**dass** eine bevorzugte Ausführung folgende Konstruktionsmerkmale aufweist:
Durch **(In /1**) **gekennzeichnet**e Stelle bezeichnet die Blutabnahme (iV) eines Patienten, bei der die Weiterleitung durch z.B. geeignete Schlauchverbindung in den (**FC / 2**) Flow-Controler (Fliessgeschwindigkeit / Pump-Geschwindigkeit). Der Flow-Controller) regelt über einen Mikroprozessor die Pump-Geschwindigkeit, wobei verschiedene Ausführungsformen denkbar sind. **(Inj** / **3)** dosiert eine konstante Menge an Anti-Koagulantien zur Vermeidung der Blutgerinnung. Das Voll-Blut wird nach dem Injektor in eine dynamische Mischkammer **(MC / 4)** geleitet um eine Sedimentation durch Mixen / Rühren auszuschliessen um dann in die "Reaktions-Zelle **(EC / 5)** zu gelangen, die temperierbar sein kann. Nach Verlassen der Reaktions-Zelle (EC) wo die Inaktivierung von resistenten Keimen / Viren geschieht wird das Blut (Flüssigkeit) in eine Entgasungs-Einheit (**DGC /6**) geleitet, die temperierbar ist. Diese Entgasungs-Einheit befreit von minimalen Restgasen (Wasserstoff, Sauerstoff, Chlor oder Ozon je nach Elektrode) die sich bei der Dekontamination bilden. Im einfachsten Fall besteht diese aus einem Kunststoff-Schlauch (Coil) von einigen Metern der für Gase durchlässig ist und im Vakuum betrieben wird. Im Anschluss nach Entgasung wird das Medium (Blut) wieder einer Mischkammer **(MC /7)** zugeführt, um eine Sedimentation zu vermeiden. **(FU /8)** ist eine optionale Filtrationseinheit mit einer optionalen Luftblasenfalle. Diese Filtrationseinheit kann aus synthetischen oder Zellulose-Material bestehen und könnte in "Cross-Flow-Verfahren" Zell-Debris entfernen. Mit gleicher Einheit könnte aber auch durch Adsorption z.B. über Nano-Material, eventuell freigesetzte Toxine (Jarrisch-Herxheimer Reaktion) gebunden werden. Danach wird unter Kennzeichnung **(Out** / **9)** dem Patienten (iV) das Blut wieder zurückgegeben.

14. Vorrichtung nach einem der vorangegangenen Ansprüche
**dadurch gekennzeichnet,**
**dass** eine Flüssigkeit mit vorzugsweise konstantem Fluss durch eine Apparatur geleitet wird die aus biokompatiblen Materialien besteht, deren Hauptmerkmal eine Reaktions-Zelle bildet, deren Gehäuse Strom nicht leitet und im Inneren des Gehäuses eine Anzahl parallel angeordneter Platten oder auch nur zwei Platten (als Art einer Elektrode mit beliebigem Abstand) enthält, die
a.) einen Stromfluss in der Flüssigkeit ermöglichen,
b.) eine Elektrolyse zur Freisetzung von chemischen Stoffen gestatten und
c.) die eine elektromagnetische Schwingung, Oszillation durch die umgebende Flüssigkeit als Kopplungsmedium übertragen um eine letale Resonanz für biologische Partikel, ermöglichen.
